# EUROPÄISCHE PATENTSCHRIFT

(11) **EP 2 543 239 B1**
(45) Veröffentlichungstag und Bekanntmachung des Hinweises auf die Patenterteilung: **15.05.2019**
(21) Anmeldenummer: 10716754.6
(22) Anmeldetag: 05.03.2010
(51) Int. Cl.: H05H 7/12, H05H 13/00, H05H 13/04, A61N 5/10

(54) **VERFAHREN ZUM BETRIEB EINER TEILCHENBESCHLEUNIGERVORRICHTUNG SOWIE TEILCHENBESCHLEUNIGERVORRICHTUNG**
METHOD FOR OPERATING A PARTICLE ACCELERATING DEVICE AND PARTICLE ACCELERATING DEVICE
PROCÉDÉ POUR FAIRE FONCTIONNER UN DISPOSITIF ACCÉLÉRATEUR DE PARTICULES ET DISPOSITIF ACCÉLÉRATEUR DE PARTICULES

(43) Veröffentlichungstag der Anmeldung: 09.01.2013
(73) Patentinhaber: GSI Helmholtzzentrum für Schwerionenforschung GmbH, 64291 Darmstadt (DE)
(72) Erfinder: FRANCZAK, Bernhard, 64293 Darmstadt (DE)
(86) Internationale Anmeldenummer: PCT/EP2010/001380
(87) Internationale Veröffentlichungsnummer: WO 2011/107120

(56) Entgegenhaltungen:
- P.J. BRYANT (ED.): "Proton-Ion Medical Machine Study (PIMMS) Part I", CERN/PS 99-010 (DI), 2. März 1999 (1999-03-02), Seiten 26-29, XP002609770, European Organization for nuclear Research; Geneva,Switzerland

## Beschreibung

Die Erfindung betrifft ein Verfahren zum Betrieb einer Teilchenbeschleunigervorrichtung. Weiterhin betrifft die Erfindung eine Teilchenbeschleunigervorrichtung zur Erzeugung beschleunigter Teilchen, insbesondere zur Erzeugung eines vorzugsweise hochenergetischen Teilchenstrahls.

Teilchenbeschleuniger werden zwischenzeitlich nicht mehr ausschließlich für Forschungszwecke, insbesondere für Forschungszwecke auf dem Gebiet der Grundlagenforschung des Aufbaus der Materie, verwendet, sondern fangen bereits an, sich auf unterschiedlichen technischen Gebieten zu etablieren. Ein Beispiel hierfür ist die Ausbildung von Mikrostrukturen (wie beispielsweise zur Herstellung hochintegrierter elektronischer Schaltkreise sowie mechanischer Nanostrukturen), die gezielte Implantierung von Fremdpartikeln (insbesondere Ionen) in tiefer gelegenen Schichten zur Ausbildung von strukturierten Halbleitermaterialien, sowie insbesondere auch der medizinische Bereich. Hier werden beschleunigte Teilchen bereits seit geraumer Zeit zur Bestrahlung von Tumoren verwendet. Derartige Teilchenstrahlen aus hochbeschleunigten Teilchen haben - neben einer Bestrahlung mit elektromagnetischen Wellen, wie speziell Röntgenstrahlen - den Vorteil, dass sie ohne Operation angewendet werden können. Dadurch wird es möglich, auch an sich nicht-operable Tumoren therapieren zu können. Eine derartige Nicht- Operierbarkeit kann beispielsweise in der Lage der Tumoren begründet liegen, beispielsweise dadurch, dass diese operativ nicht erreicht werden können beziehungsweise bei einer Operation eine größere Menge an kritischem Gewebe zerstört werden müsste. Dies betrifft beispielsweise in besonderem Maße Gehirntumoren.

Ein Vorteil bei der Verwendung von Teilchenstrahlen gegenüber der Verwendung von elektromagnetischen Strahlen (wie beispielsweise Röntgenstrahlen) besteht darin, dass Teilchenstrahlen ihre Energie nicht gleichmäßig über ihre Flugbahn hinweg abgeben. Stattdessen ist es für Teilchenstrahlen typisch, dass sie ihre Energie auf ihrem Weg durch die von ihnen durchlaufene Materie hindurch in gewissen Bereichen in stärkerem Maße abgeben, als dies in anderen Bereichen der Fall ist. Dieses Verhalten ist bei unterschiedlichen Teilchenarten unterschiedlich stark gegeben. Besonders ausgeprägt ist diese "selektive" Energieabgabe über eine vergleichsweise kurze Wegstrecke hinweg bei Hadronen, speziell bei Ionen, und hier insbesondere bei schweren Ionen wie insbesondere Kohlenstoffionen. Insbesondere bei Schwerionen erfolgt die "selektiv" Energieabgabe am Ende der Reichweite. Hier ist die Energieabgabe auf eine sehr kurze Strecke konzentriert, der als "Bragg-Peak" bezeichnet wird. Mit einer geeigneten Fokussierung eines Schwerionenstrahls in lateraler Richtung in Verbindung mit einer passend gewählten Teilchenenergie werden dadurch Bestrahlungsvolumen im Bereich von einigen wenigen Kubikmillimetern möglich (wobei Bestrahlungsvolumen von lediglich 1 mm³ als technologisch möglich erscheinen). Bereits diese Zahl macht das große Potenzial der Verwendung von Schwerionenstrahlen bei der Tumortherapie offensichtlich.

Aus diesem Grunde wurden trotz des erheblichen technischen Aufwands bereits erste Teilchenbeschleuniger die ausschließlich der Tumortherapie dienen errichtet, beziehungsweise werden aktuell gebaut. In Deutschland wird derzeit beispielsweise in Marburg ein Therapie-Synchrotron gebaut, während eine Anlage in Heidelberg vor kurzem fertiggestellt wurde und bereits mit dem Therapiebetrieb begonnen hat. Die ersten bereits im Betrieb befindlichen Anlagen sowie Forschungs-Synchrotrons, die zeitweise zu Zwecken der Tumortherapie verwendet wurden, haben sehr positive Therapieresultate erzielen können.

Der Betrieb heutiger Therapie-Synchrotrons erfolgt in aller Regel unter Verwendung sogenannter Raster-Scanning-Verfahren. Dabei wird der aus dem Synchrotron extrahierte Schwerionenstrahl kurz vor dem Zielgebiet (also dem Patienten beziehungsweise dem Therapieraum) durch so genannte Scanning-Magnete geleitet. Diese lenken den Teilchenstrahl in der X-Y-Ebene im Zielgebiet ab (wobei die Tiefenlage der Ziel-Ebene durch die Energie des Teilchenstrahls sowie die Art, Lage und Menge des in Strahlrichtung gesehen vor dem Zielgebiet liegenden Gewebes beziehungsweise andere Materialien bestimmt wird). Im Therapiebetrieb werden üblicherweise die einzelnen Punkte der Ebene sukzessive nacheinander angefahren. Dabei wird in jedem vom Teilchenstrahl angefahrenen, kleinen Zielvolumen (üblicherweise als "Rasterpunkt" oder "Voxel" bezeichnet) eine bestimmte Dosis, die im Rahmen einer so genannten Bestrahlungsplanung vorab berechnet wird, deponiert. Die Dosismenge kann beispielsweise durch eine entsprechend lange zeitliche Beaufschlagung des entsprechenden Voxels mit dem Teilchenstrahl erzielt werden. Werden zusätzlich unterschiedliche X-Y-Ebenen (Isoenergieebenen) durch eine Variation der Teilchenenergie "durchgefahren", so ist es möglich, ein beliebiges dreidimensionales Gebilde gezielt bestrahlen zu können. Dadurch kann die zerstörende Wirkung des Teilchenstrahls sehr genau auf einen auch unregelmäßig geformten Tumor konzentriert werden, wobei umliegendes gesundes Gewebe geschont wird.

Ein "Durchfahren" von in unterschiedlichen Tiefen gelegenen Ebenen (also eine Variation des Zielgebiets in Z-Richtung) erfolgt dadurch, dass die Energie des Teilchenstrahls geändert wird. Dazu sind im Stand der Technik zwei unterschiedliche Verfahren bekannt:
Bei einem ersten Verfahren wird ein Teilchenstrahl mit stets gleicher Teilchenenergie (zumindest während eines Behandlungsvorgangs) erzeugt. Dieser Teilchenstrahl wird, bevor er auf den Patienten trifft, durch ein energieabsorbierendes Material geleitet. Dieses energieabsorbierende Material weist eine nicht-konstante Dicke auf und ist beispielsweise keilförmig ausgebildet. Durch eine geeignete Verschiebung des energieabsorbierenden Materials muss der Teilchenstrahl eine unterschiedliche Länge durch das energieabsorbierende Material hindurch laufen, bevor er schlussendlich auf den Patienten trifft. Dadurch kann die Energie des beim Patienten ankommenden Teilchenstrahls gezielt verändert werden. Ein großes Problem dabei ist einerseits der mechanische Aufwand der Steuerung der Verschiebung, insbesondere aber auch die Verschlechterung der Güte des beim Patienten ankommenden Teilchenstrahls. So weitet sich der Teilchenstrahl typischerweise deutlich auf (üblicherweise nimmt der Durchmesser des Teilchenstrahls um einen Faktor 2 oder mehr zu). Um dieser Verschlechterung der Eigenschaft des Teilchenstrahls entgegenzuwirken wären nachgeschaltete Fokussierungsmagnete erforderlich, die jedoch teuer sind und insbesondere in aller Regel die ursprüngliche Qualität des Teilchenstrahls nicht wiederherstellen können. Darüber hinaus kommt es beim Durchlaufen von Materie üblicherweise zu einer Energiestreuung, und damit effektiv zu einer Verbreiterung des Bragg-Peaks, also ebenfalls zu einer Verschlechterung des Teilchenstrahls. Zur "Korrektur" der hierdurch induzierten Fehler wären Energiefilter erforderlich, die nicht nur entsprechend aufwändig sind, sondern oftmals auch die Anzahl der für die eigentliche Therapie zur Verfügung stehenden Teilchen zum Teil stark vermindern. Eine Variation des beschriebenen Verfahrens besteht darin, dass das energieabsorbierende Material starr platziert ist, und der Teilchenstrahl durch eine geeignete Ablenkung an unterschiedliche Orte des energieabsorbierenden Materials gelenkt wird.

Eine andere Methode um die Teilchenenergie zu verändern besteht darin, dass die Energie der Teilchen, auf die sie im Synchrotron beschleunigt werden, unterschiedlich hoch gewählt wird. So ist es bei einem Teil der heutzutage vorhandenen Synchrotrons möglich, dass die Teilchenenergie von einem auf den anderen Beschleunigungszyklus geändert werden kann. Während eines einzelnen Extraktionszyklus (der bei einer langsamen Extraktion durchaus im Bereich zwischen 3 und 10 Sekunden andauern kann) bleibt die Energie der Teilchen dagegen konstant. Für die Therapie eines unbeweglichen Tumors stellt dies üblicherweise kein relevantes Problem dar. Die Zeitdauer für die Behandlung einer einzelnen X-Y-Ebene erfordert nämlich in der Regel mehrere Teilchenextraktionszyklen. Allenfalls beim "letzten" Extraktionszyklus beim Durchscannen einer X-Y-Ebene kann es vorkommen, dass die noch im Synchrotron verbliebenen umlaufenden Ionen verworfen werden müssen. Insgesamt beträgt der hierdurch bedingte Verlust an effektiver Strahlzeit jedoch nur einen kleinen Anteil an der Gesamtbehandlungsdauer.

Derartige Beschleunigervorrichtungen nach dem Stand der Technik sind beispielsweise in US 6,903,351B1, US 2002/0014588 A1 oder DE 10 2005 015 601 A1 beschrieben.

Das Dokument "Proton Ion Medical Machine Study (PIMMS) Part I" (P.J. Bryant (Ed.), CERN /PS 99-010 (D1)) beschreibt einen Synchrotron-Beschleuniger für die Bestrahlung von Tumorpatienten mittels eines Rasterscan-Verfahrens, wobei ein lonenstrahl durch langsame Resonanz-Extraktion aus dem Synchrotron erzeugt wird.

Ein großes Problem bei den heute verfügbaren Teilchenbeschleunigern stellt jedoch nach wie vor die Therapie von sich bewegenden Tumoren dar. Tumore bewegen sich beispielsweise, weil sie sich innerhalb oder in der Nähe von sich bewegenden Organen befinden. Hierbei kann es sich beispielsweise um das Herz (Herzschlag), die Lunge (Atembewegung) oder den Darm (Darmperistaltik) handeln. Um auch derartige, sich bewegende Tumore behandeln zu können, wurden ebenfalls unterschiedliche Verfahren vorgeschlagen, die jedoch derzeit noch zum Teil gravierende Probleme aufweisen.

So existieren beispielsweise Lösungen, bei denen gezielt ein Bereich bestrahlt wird, der größer ist, als es der eigentlichen Tumorausdehnung entspricht. Die Größe des bestrahlten Bereichs wird dabei so gewählt, dass der Tumor im zeitlichen Mittel mit einer ausreichenden Teilchendosis beaufschlagt wird. Problematisch ist es jedoch, dass hierbei zwangsweise gesundes Gewebe in Mitleidenschaft gezogen wird (der so genannte Sicherheitssaum). Während dies bei manchen Körperregionen gegebenenfalls vertretbar ist, so scheidet dieses Verfahren aus, wenn sich der (insbesondere in beweglichen Gewebe befindliche) Tumor in der Nähe von kritischem Gewebe (wie beispielsweise benachbart zu Nervenknoten, in der Nähe des Rückenmarks oder dergleichen) befindet. Ebenfalls ist dieses Verfahren problematisch, wenn die Bewegung des Tumors relativ groß ist, insbesondere groß im Verhältnis zu seiner Ausdehnung ist. Dies kann jedoch bei Lungentumoren oder Herztumoren sehr schnell der Fall sein.

Ein weiteres, bereits vorgeschlagenes Verfahren, um das Problem einer Tumorbewegung in den Griff zu bekommen, ist das so genannte "Gating". Hier wird die jeweils aktuelle Lage des zu behandelnden Tumors verfolgt. Immer dann wenn sich der Tumor im aktuell bestrahlten Zielvolumen befindet, wird der Teilchenstrahl freigegeben. Befindet sich der Tumor jedoch außerhalb des aktuell bestrahlten Zielvolumens, so wird der Teilchenstrahl unterbrochen. Wie man leicht nachvollziehen kann, führt ein derartiges Verfahren zu einer deutlichen Verlängerung der Behandlungsdauer. Dies führt bei einem ohnehin recht teuren Behandlungsverfahren zu einer überdurchschnittlich hohen, zusätzlichen Kostenerhöhung der Therapie.

Eine weitere Möglichkeit besteht darin, dass die bereits oben erwähnten, unterschiedlich dicken Elemente aus energieabsorbierendem Material genutzt werden und korrespondierend zur Bewegung des Tumors kontinuierlich nachgesteuert werden. Dies stellt in der Regel die bislang sinnvollste Therapiemöglichkeit für bewegte Tumoren dar. Die vorstehend beschriebenen Probleme sind jedoch nach wie vor vorhanden. Darüber hinaus erweist sich eine ausreichend schnelle Nachsteuerung der Position des energieabsorbierenden Materials beziehungsweise der Ablenkung des Teilchenstrahls in der Regel als aufwändig und problematisch

Die Aufgabe der Erfindung besteht somit darin, ein gegenüber dem Stand der Technik verbessertes Verfahren zum Betrieb einer Teilchenbeschleunigervorrichtung vorzuschlagen. Weiterhin besteht die Aufgabe der Erfindung darin, eine gegenüber dem Stand der Technik verbesserte Teilchenbeschleunigervorrichtung vorzuschlagen.

Ein Verfahren gemäß dem unabhängigen Verfahrensanspruch beziehungsweise eine Teilchenbeschleunigervorrichtung gemäß dem unabhängigen Vorrichtungsanspruch löst diese Aufgabe.

Dabei wird ein Verfahren zum Betrieb einer Teilchenbeschleunigervorrichtung vorgeschlagen, die einen Synchrotronring mit einzelnen Komponenten, die für eine langsame Extraktion angeordnet sind; aufweist, bei dem die Energie der von der Teilchenbeschleunigervorrichtung freigesetzten Teilchen während der Teilchenextraktionsphase durch Veränderung der Energie der Teilchen in der Teilchen¬beschleuniger¬vorrichtung zumindest zeitweise und/oder zumindest teilweise zumindest quasi-kontinuierlich verändert wird. Insbesondere zu Zeiten der quasi-kontinuierlichen Veränderung der Energie der von der Teilchenbeschleunigervorrichtung freigesetzten Teilchen ist es auf besonders einfache Weise möglich, der Bewegung eines sich bewegenden Tumors zu folgen. Dies betrifft insbesondere eine Bewegung des Tumors beziehungsweise einen Bewegungsanteil einer Bewegung des Tumors in einer parallel zur Teilchenstrahlrichtung verlaufenden Richtung. Natürlich ist es auch möglich, mit dem vorgeschlagenen Verfahren eine schnellere Veränderung der aktuell durch Rasterscanning bestrahlten X-Y-Ebene (Isoenergieebene) bewirken zu können. Durch die quasi-kontinuierliche Veränderung der Energie
der Teilchen, kann die Lage des Bragg-Peaks im Tumor verändert werden. Hierdurch kann eine Beschleunigung eines Therapievorgangs bewirkt werden. Darüber hinaus ist es nicht erforderlich, eventuell in der Teilchenbeschleunigervorrichtung verbliebene Teilchen mit einer "unpassenden" Energie nutzlos beseitigen zu müssen, was nicht nur einen erhöhten Energiebedarf, sondern insbesondere auch eine an sich nutzlose Erzeugung von radioaktiv strahlenden Substanzen zur Folge haben kann. Diese Vorteile können insbesondere dann signifikant sein, wenn das zu bestrahlende Zielgebiet (beispielsweise ein Tumor) eine nur geringe räumliche Ausdehnung in der X-Y-Ebene (Isoenergieebene) aufweist, so dass bereits nach der Beaufschlagung einiger weniger Voxel der Teilchenstrahl hinsichtlich seiner Eindringtiefe geändert werden sollte.

Unter einer quasi-kontinuierlichen Veränderung der Energie der von der Teilchenbeschleunigervorrichtung freigesetzten Teilchen ist insbesondere zu verstehen, dass die Energie nur in gewissen Zeiträumen "frei" veränderlich ist, weil beispielsweise Teilchen nur in bestimmten Zeitintervallen freigegeben werden. Im Falle von Synchrotrons wechseln Teilcheninjektionsphasen, Teilchenbeschleunigungsphasen und Teilchenextraktionsphasen einander ab. Wenn die Energie der freigesetzten Teilchen nur während der Extraktionsphasen (beziehungsweise während Teilen davon) "frei" verändert werden kann, so kann dies einer quasi-kontinuierlichen Energievariation im Sinne dieser Anmeldung entsprechen. Weiterhin kann der Begriff der quasi-kontinuierlichen Energievariation auch eine stufenartige Variation der Energie der von der Teilchenbeschleunigervorrichtung freigesetzten Teilchen umfassen. Dies kann sich insbesondere auf eine bestimmte Zeit-Taktrate und/oder eine bestimmte Stufung der Energie beziehen. Die Zeit-Taktrate beziehungsweise die Energiestufung kann dabei insbesondere feste und/oder variable Zeitschritte beziehungsweise Energieschritte aufweisen. Die "freie" Veränderbarkeit der Teilchenenergie kann natürlich auf Energieintervalle mit einer bestimmten Größe beschränkt sein. Beispielsweise kann
sich die Veränderbarkeit auf ±1 %, ±2 %, ±3 %, ±4 %, ±5 %, ±6 %, ±7 %, ±8 %, ±9 % und/oder ±10 % des "typischen" Energiewerts der Teilchenbeschleunigervorrichtung beschränken. Auch asymmetrisch liegende Energieintervalle sind selbstverständlich denkbar, wobei die oben genannten Zahlenwerte in im Wesentlichen beliebiger Weise miteinander kombiniert werden können.

Bei bisherigen Teilchenbeschleunigervorrichtungen wurde eine Energieanpassung - falls überhaupt - bislang nur zwischen zwei Beschleunigungsintervallen und damit bestenfalls zyklisch durchgeführt. Bei einem Synchrotron erfolgt ein Wechsel der Teilchenenergie also bestenfalls von einem Extraktionszyklus auf den nächsten Extraktionszyklus, während der eigentliche Extraktionszyklus jeweils mit konstanter Teilchenenergie erfolgt. Dieses Vorgehen wurde bislang als technisch zwangsläufig erforderliches Vorgehen angesehen. Die umlaufenden Teilchenpakete weisen nämlich während der eigentlichen Beschleunigungsphase im Synchrotron eine relativ hohe Eigenstabilität auf. Dadurch haben kleinere, technisch üblicherweise nicht zu verhindernde Schwankungen bei den verwendeten Dipolmagnetfeldern, bei den verwendeten Quadrupolmagnetfeldern sowie bei den beschleunigenden elektrischen Hochfrequenzfeldern üblicherweise keinen besonders nachteiligen Einfluss auf den umlaufenden Teilchenstrahl (wie beispielsweise einen Teilchenstrahlverlust). Während der Extraktionsphase wird jedoch der umlaufende Teilchenstrahl durch so genannte Exciter-Magnete (insbesondere so genannte KO-Exciter) künstlich in eine Schwingungsanregung versetzt, so dass Anteile des beziehungsweise der umlaufenden Teilchenpakete aus dem Synchrotronring extrahiert werden können und in Richtung des mit dem Teilchenstrahl zu beaufschlagenden Werkstücks beziehungsweise Patienten gerichtet werden können. Grundsätzlich sind aber auch andere Extraktionsverfahren, wie beispielsweise eine Q-Wert-Verschiebung denkbar. In dieser Phase ist der Teilchenstrahl sehr empfindlich gegenüber Störungen, so dass es bei Schwankungen bei der Ansteuerung des Synchrotronrings leicht zu einem Strahlverlust kommen kann. Es wurde daher bislang davon ausgegangen, dass eine Veränderung der Energie der umlaufenden Teilchen, die eine damit einhergehende Anpassung der unterschiedlichen Magnetfelder (zum Beispiel der Dipolmagnete und/oder der Quadrupolmagnete) und der unterschiedlichen elektrischen Felder (zum Beispiel der Beschleunigungsfelder) erfordert, tunlichst zu vermeiden ist, da dies zu einem inakzeptabel häufigen Strahlverlust führen würde. Die Erfinder haben jedoch festgestellt, dass bei einer geeigneten Ansteuerung der Vorrichtung diese Probleme - entgegen dem derzeit gültigen Paradigma im Beschleunigerbau - beherrschbar sind und die Vorteile die Nachteile bei weitem überwiegen können.

Vorteilhaft ist es, wenn die Teilchenbeschleunigervorrichtung derart eingerichtet ist, dass die Teilchensorte (beispielsweise zyklisch) geändert werden kann (beispielsweise von Heliumionen auf Kohlenstoffionen) und/oder auch Gemische von unterschiedlichen Teilchen (beispielsweise Gemische aus Heliumionen und Kohlenstoffionen) erzeugt, beschleunigt und ausgegeben werden können. Insbesondere im Fall von zyklischen (zyklisch arbeitenden) Teilchenbeschleunigervorrichtungen, wie beispielsweise Synchrotrons, ist es vorteilhaft, wenn die Teilchensorte von einem Beschleunigungsintervall auf das nächste geändert werden kann (beispielsweise ein Beschleunigungsdurchgang mit Kohlenstoffionen, und ein daran anschließender Beschleunigungsdurchgang mit Sauerstoffionen). Unter einem Wechsel der lonensorte kann im Übrigen auch die Veränderung eines Mischungsverhältnisses bei Nutzung eines Teilchengemischs verstanden werden (beispielsweise im Falle eines Gemischs aus Heliumionen und Kohlenstoffionen eine Veränderung der Anteile von Heliumionen zu Kohlenstoffionen, insbesondere von einem Beschleunigungsdurchgang zum nächsten hin).

Eine vorteilhafte Weiterbildung des Verfahrens kann sich ergeben, wenn es sich bei der Teilchenbeschleunigervorrichtung um eine Teilchenbeschleunigervorrichtung für Hadronen, insbesondere für Protonen, für Pionen, für Ionen und/oder für Schwerionen handelt. Eine Teilchenbeschleunigervorrichtung für Pionen (gegebenenfalls auch für andere Teilchen) wird dabei in der Regel derart realisiert, dass beispielsweise Protonen beschleunigt werden, und die Pionen erst "am Ende" nach dem Beschleunigungsvorgang erzeugt werden. Üblicherweise können mit zyklischen Teilchenbeschleunigervorrichtungen Teilchenstrahlen mit Teilchen, die eine höhere Energie aufweisen, erreicht werden. Die Energie eines derartigen Teilchenstrahls liegt in der Größenordnung etwa von einem GeV bis zu einigen zehn GeV. Dadurch ist es möglich, auch relativ tief, beispielsweise einige Zentimeter im Gewebe eines Patienten sitzende Zielvolumen behandeln zu können. Entsprechendes gilt für mit einer Teilchenstrahlung zu beaufschlagende Materialien, wie beispielsweise bei der Nanostrukturierung von Werkstoffen, oder bei der gezielten Implantierung von Ionen in tiefliegende Schichten eines (Halbleiter-) Materials. Unter einer "zyklischen Teilchenbeschleunigervorrichtung" ist dabei insbesondere zu verstehen, dass die Teilchen in der Teilchenbeschleunigervorrichtung zumindest zeitweise und/oder zumindest teilweise zyklisch umlaufen und damit ein bestimmter Streckenabschnitt der Teilchenbeschleunigervorrichtung zumindest zeitweise und/oder zumindest teilweise ein bestimmtes Teilchen beziehungsweise ein bestimmtes Teilchenpaket mehrfach durchlaufen wird und gegebenenfalls das Teilchen bzw. Teilchenpaket mehrfach beschleunigt wird. Lediglich der Vollständigkeit halber wird darauf hingewiesen, dass auch zyklische Teilchenbeschleunigervorrichtungen üblicherweise in bestimmten Bereichen lineare Teilchenbeschleunigervorrichtungen aufweisen. Wenn es sich um ein Synchrotron handelt, so weist dieses in aller Regel neben einer lonenquelle zunächst einen Linearbeschleuniger auf, der die Teilchen initial beschleunigt, bevor sie in den eigentlichen Synchrotronring injiziert werden.

Ein weiterer Vorteil von zyklischen Teilchenbeschleunigervorrichtungen besteht in der Regel darin, dass in diesen auch bereits hoch beschleunigte Teilchen über relativ lange Zeiträume zwischengespeichert werden können. Dadurch ist es möglich, die beschleunigten Teilchen relativ langsam aus der Speichervorrichtung zu extrahieren um einen Gegenstand, beispielsweise einen Patienten damit zu beaufschlagen. Dies kann sich als wichtig erweisen, beispielsweise um einen Patienten nicht mit einem unnötig hohen beziehungsweise übermäßig hohen Teilchenfluss zu beaufschlagen. Vorteilhaft ist es darüber hinaus, wenn es sich bei der Teilchenbeschleunigervorrichtung um eine Teilchenbeschleunigervorrichtung für Hadronen handelt. Hadronen weisen üblicherweise einen besonders ausgeprägten Bragg-Peak auf, so dass eine genauere Lokalisierung der beaufschlagten Dosisleistung in Z-Richtung (Richtung parallel zum Teilchenstrahl) möglich ist. Bei den Hadronen kann es sich insbesondere um Protonen, Pionen, Ionen und Schwerionen handeln. Unter Schwerionen werden üblicherweise Ionen mit einer Massenzahl von zumindest 2, 3, 4, 5, 6, 7, 8, 9 oder 10 verstanden. Besonders bewährt haben sich für die Behandlung von Tumoren insbesondere Kohlenstoffionen, Neonionen sowie Sauerstoffionen. Lediglich der Vollständigkeit halber wird darauf hingewiesen, dass Ionen üblicherweise einfacher zu beschleunigen sind. Darüber hinaus haben die bereits durchgeführten Therapien gezeigt, dass auch die lonenladung einen Einfluss auf den Therapieerfolg haben kann. Dementsprechend ist es sinnvoll, die Ionenladung auf die jeweilige Therapie geeignet anzupassen.

Das Verfahren wird derart durchgeführt, dass die Energie der von der Teilchenbeschleunigervorrichtung freigesetzten Teilchen zumindest zeitweise und/oder zumindest teilweise während der Teilchenextraktion verändert wird. Mit anderen Worten kann die Anpassung der Teilchenenergie quasi zeitgleich mit der Nutzung der beschleunigten Teilchen erfolgen. Dadurch kann eine besonders gute Übereinstimmung zwischen erwünschter und tatsächlicher Teilchenenergie realisiert werden. Darüber hinaus können unnötige Beschleunigungs- beziehungsweise Verzögerungsvorgänge vermieden werden, die zu einem unnötigen Energieverbrauch und/oder zu einer Verschlechterung der Teilchenstrahlqualität führen könnten. Es sollte darauf hingewiesen werden, dass insbesondere ein Abbremsen von Teilchen speziell bei zyklischen Teilchenbeschleunigervorrichtungen (wie einem Synchrotron) in der Regel ohne Probleme und ohne gesonderte Vorrichtungen möglich ist, da die Teilchen, dadurch dass sie auf eine Kreisbahn gezwungen werden, Energie in Form der sogenannten Synchrotronstrahlung abstrahlen (wobei die Synchrotronstrahlung bei Schwerionen im Verhältnis zu beispielsweise Leptonen relativ klein ist). Dieser Energieverlust entspricht einem Abbremsen der Teilchen.

Eine besonders vorteilhafte Ausgestaltung des Verfahrens ergibt sich, wenn die Teilchen zumindest zeitweise und/oder zumindest teilweise in einem ersten Schritt auf einen Energie-Initialwert beschleunigt werden und in einem zweiten Schritt die Teilchen auf einen Energie-Sollwert beschleunigt und/oder verzögert werden. Wie bereits erwähnt, ist beispielsweise bei einem Synchrotronring der umlaufende Teilchenstrahl deutlich eigenstabiler, solange (noch) keine Extraktion von Teilchen erfolgt. Dadurch ist es möglich, höhere Toleranzen von einzelnen Komponenten tolerieren zu können. Dadurch wiederum ist es möglich, den eigentlichen, initialen Beschleunigungsvorgang besonders schnell durchführen zu können. Insbesondere kann es bei einem derartigen initialen Beschleunigungsvorgang aufgrund von Induktionseffekten bei den verwendeten Magneten (insbesondere Dipolmagnete und/oder Quadrupolmagnete) zu gewissen Schwankungen der erzeugten Magnetfelder kommen. Darüber hinaus kann die initiale Beschleunigung unter Verwendung von Ansteuerungsalgorithmen erfolgen, die ohne beziehungsweise mit einem geringeren Einfluss von zu messenden Messwerten (beispielsweise gemessene Magnetfelder oder dergleichen) auskommen. Auch dadurch kann die initiale Beschleunigungsphase zum Teil erheblich verkürzt werden. Insbesondere können auch Probleme durch "langsame" Messgeräte vermindert oder sogar verhindert werden. Erst sobald der anfängliche Energie-Initialwert erreicht wird, werden die Energien auf den eigentlichen Energie-Sollwert gebracht. Dieser Anpassvorgang ist üblicherweise langsamer durchzuführen (insbesondere aus den vorerwähnten Gründen). Durch die vorgeschlagene zweistufige Beschleunigung der Teilchen kann somit im zeitlichen Mittel Zeit eingespart werden. Vorteilhafterweise wird der Energie-Initialwert derart gewählt, dass er in der Nähe der zu erwartenden Energie-Sollwerte liegt und vorzugsweise in der Nähe des zu erwartenden Energiemittelwerts der zu erwartenden Energie-Sollwerte liegt. Der Übergang zwischen der initialen Beschleunigung auf den Energie-Initialwert und der Nachbeschleunigung (beziehungsweise Nachverzögerung) auf den eigentlichen Energie-Sollwert kann in beliebiger Weise nahtlos und/oder nach Ablauf einer bestimmten Pausenzeit durchgeführt werden. Insbesondere im letzten Fall können Einschwingvorgänge (insbesondere solche, die durch induktive Effekte bedingt sind) abgewartet werden. Dies kann die Qualität des Teilchenstrahls erhöhen und/oder die Wahrscheinlichkeit für einen Teilchenstrahlverlust verringern. Mit einer "zeitlichen Pause" zwischen dem Erreichen des Energie-Initialwerts und (dem Beginn) der Anpassung auf einen Energie-Sollwert kann auch die Zeitspanne überbrückt werden, die erforderlich ist, dass sich im Falle eines sich bewegenden Zielvolumens (zum Beispiel eines sich bewegenden Tumors) das Zielvolumen zum Beispiel erstmals an einem für den Energie-Initialwert "passenden" Ort befindet.

Vorteilhaft ist es weiterhin, das Verfahren derart auszugestalten, dass die Energie der von der Teilchenbeschleunigervorrichtung freigesetzten Teilchen zumindest zeitweise und/oder zumindest teilweise in Abhängigkeit von einem Sollwert und/oder in Abhängigkeit von einem Messwert verändert wird, bevorzugt in Abhängigkeit von einer erforderlichen Eindringtiefe in ein zu bestrahlendes Werkstück und/oder in Abhängigkeit von der Lage eines zu bestrahlenden, insbesondere sich bewegenden Zielvolumens eines zu bestrahlenden Werkstücks verändert wird. Der Sollwert kann dabei beispielsweise von einer Steuerschaltung generiert werden. Die Steuerschaltung kann dabei insbesondere auch einen Messwert beziehungsweise mehrere Messwerte nutzen, um das Sollwertsignal (zum Beispiel ein Energiesollwertsignal) zu generieren. Dadurch kann eine besonders genaue, insbesondere zielgenaue Bestrahlung eines Zielvolumens (zum Beispiel eines einzelnen Rasterpunkts) erfolgen, was entsprechend vorteilhaft ist. Durch die Variation der Teilchenenergie in Abhängigkeit von einer erwünschten Eindringtiefe in ein zu bestrahlendes Werkstück, kann eine besonders rasche dreidimensionale Durchrasterung oder Abtastung eines Zielvolumens in dem Werkstück realisiert werden. Wird die Teilchenenergie in Abhängigkeit von der Lage eines zu bestrahlenden, insbesondere sich bewegenden Zielvolumens in einem zu bestrahlenden Werkstück geändert, so ist es möglich, auch ein sich beispielsweise stark und/oder ein sich relativ schnell bewegendes Zielvolumen besonders genau mit einer erwünschten Dosis zu beaufschlagen. Bei dem "Werkstück" kann es sich insbesondere auch um einen Patienten handeln. Speziell in diesem Fall kann es sich bei dem bewegten Zielvolumen beispielsweise im Wesentlichen um einen zu bestrahlenden Tumor handeln (wobei selbstverständlich auch Sicherheitssäume und dergleichen mit berücksichtigt werden können). Beispielsweise ist dadurch die Therapie von Tumoren möglich, die sich zum Beispiel in (oder unmittelbar benachbart zu) der Lunge oder dem Herz befinden. Darüber hinaus kann auch umliegendes (insbesondere kritisches) Material beziehungsweise Gewebe bei der Bestrahlung besonders gut geschont werden. Insbesondere ist durch eine entsprechende Strahlnachführung (insbesondere hinsichtlich der Energie) auch eine fortdauernde Beaufschlagung eines definierten Rasterpunkts oder Voxels möglich, selbst wenn sich dieser im Raum bewegt. Lediglich der Vollständigkeit halber wird darauf hingewiesen, dass bei einer Beaufschlagung eines sich bewegenden Zielvolumens üblicherweise nicht nur die Eindringtiefe des Teilchenstrahls (Z-Richtung), sondern auch die laterale Lage des Teilchenstrahls (X-Y-Richtung; Ablenkung innerhalb einer Isoenergieebene), beispielsweise unter Verwendung einer Scannervorrichtung, nachgeführt werden muss.

Eine weitere besonders bevorzugte Ausgestaltung des Verfahrens ergibt sich, wenn die Lage und/oder die Bewegung eines zu bestrahlenden, insbesondere sich bewegenden Zielvolumens mit einem Extrapolationsalgorithmus vorab bestimmt wird. Durch diese zeitlich vorgezogene Bestimmung ist es möglich, den Teilchenstrahl (einschließlich des Bragg-Peaks) besonders genau einem sich bewegenden Rasterpunkt oder Voxel folgen zu lassen. Dieser erwartete Bewegungsverlauf kann beispielsweise als Rechenbasis für die erforderliche Energienachführung und/oder Scanner-Nachführung des Ionenstrahls herangezogen werden. Der "zeitliche Vorlauf", der durch diese Vorab-Bestimmung erzielt wird, sollte möglichst in etwa den Zeitdauern entsprechen, die für die erforderlichen Rechenschritte und Steuersignalerzeugungen zur eigentlichen Nachführung des lonenstrahls (Energienachführung und/oder Ortsnachführung) erforderlich sind. Typische Zeitdauern hierfür sind 10-30 Millisekunden, insbesondere 15-25 Millisekunden, besonders bevorzugt 20 Millisekunden. Die Extrapolation kann beispielsweise unter Verwendung von linearen Extrapolationsalgorithmen, quadratischen Extrapolationsalgorithmen, kubischen Extrapolationsalgorithmen und/oder Spline-Extrapolationsalgorithmen realisiert werden. Die Extrapolation und/oder eine gegebenenfalls verwendete Interpolation (insbesondere eine Interpolation zwischen zwei berechneten, extrapolierten Werten) kann beispielsweise durch Addition oder Subtraktion entsprechender Korrekturterme auf den jeweils aktuellen Wert erfolgen.

Eine weitere vorteilhafte Weiterbildung des Verfahrens ergibt sich, wenn zumindest ein Verifikationsmittel vorgesehen ist, welches überprüft, ob eine angeforderte Teilchenenergie und/oder eine angeforderte Veränderungsrate der Teilchenenergie noch innerhalb der Betriebsparameter der Teilchenbeschleunigervorrichtung liegt. Das Verifikationsmittel kann dabei in beliebiger Weise ausgeführt sein und insbesondere softwaremäßig und/oder hardwaremäßig implementiert werden. Wie bereits erwähnt, ist bei Verwendung eines Synchrotrons der Teilchenstrahl während einer Extraktionsphase relativ empfindlich, so dass zur Vermeidung von magnetischen und/oder elektromagnetischen Feldschwankungen und dergleichen, die zu einem Verlust des Teilchenstrahls führen könnten, die Teilchenenergie nur relativ langsam nachgeführt werden kann. Sollte sich beispielsweise das Zielvolumen zu schnell bewegen, kann das Verifikationsmittel ein entsprechendes Signal (beispielsweise Signal-Flag) ausgeben. Beim Auftauchen eines derartigen Signals kann beispielsweise die Extraktion des Teilchenstrahls (kurzzeitig) ausgesetzt werden. Möglich ist es auch, dass beim (insbesondere wiederholten) Auftauchen eines derartigen Signals auf eine alternative Bestrahlungstechnik oder dergleichen umgestellt wird.

Weiterhin ist es vorteilhaft das Verfahren derart durchzuführen, dass insbesondere im Bereich eines Zielvolumens zumindest zeitweise und/oder zumindest teilweise eine laterale Veränderung des von der Teilchenbeschleunigervorrichtung erzeugten Teilchenstrahls erfolgt und/oder ein eindimensionales, zweidimensionales oder dreidimensionales Scanningverfahren (speziell Raster-Scanning-Verfahren) durchgeführt wird. Auf diese Weise ist es nicht nur möglich, eine Variation des Bestrahlungsorts in Z-Richtung (Richtung parallel zum Teilchenstrahl) zu realisieren (insbesondere durch Variation des Schwerpunkts der Energieabgabe aufgrund des Bragg-Peaks), sondern vielmehr auch eine Variation des Bestrahlungsorts in einer Ebene, welche in Wesentlichen senkrecht zur Teilchenstrahlrichtung (beziehungsweise in einer Isoenergieebene) liegt. Dadurch ist es insbesondere möglich, beliebige (quasi-) zweidimensionale und/oder dreidimensionale Zielvolumen durchzuscannen, insbesondere unter Verwendung eines Raster-Scanverfahrens, bei dem sukzessive einzelne, innerhalb des Zielvolumens liegende Voxel angesteuert werden.

Eine weitere vorteilhafte Weiterbildung des Verfahrens ergibt sich, wenn das Verfahren zur Ansteuerung einer Vorrichtung verwendet wird, welche der Strahlentherapie, insbesondere der Strahlentherapie von Tumoren, bevorzugt der Strahlentherapie von sich bewegenden Tumoren dient. Mit einem derartigen Verfahren beziehungsweise einer derartigen Vorrichtung lassen sich, wie bereits vorab beschrieben, Tumoren besonders gut und effektiv therapieren. Dies gilt insbesondere auch für an sich inoperable Tumore und/oder Tumore, welche sich in oder benachbart zu sich bewegenden Gewebeteilen, wie insbesondere sich bewegenden Organen (beispielsweise Darm, Lunge oder Herz) befinden. Gerade für derartige Tumore sind bisherige Therapieverfahren oftmals noch unzureichend.

Weiterhin wird eine Teilchenbeschleunigervorrichtung zur Erzeugung beschleunigter Teilchen, insbesondere zur Erzeugung eines vorzugsweise hochenergetischen Teilchenstrahls vorgeschlagen, die einen Synchrotronring mit einzelnen Komponenten, die für eine langsame Extraktion angeordnet sind, umfasst, und die außerdem eine Steuervorrichtung umfasst, um zumindest zeitweise und/oder zumindest teilweise während einer Teilchenextraktionsphase (III) ein Energie-Sollsignal auszugeben, zu verarbeiten und/oder zu empfangen, wobei die Steuervorrichtung eine Speichervorrichtung zur Speicherung von Geräteparametern in Abhängigkeit des Energie-Sollsignals aufweist, wobei die Steuervorrichtung zur Steuerung der einzelnen Komponenten so eingerichtet ist, dass durch Veränderung der Energie der Teilchen in der Teilchenbeschleunigervorrichtung während der Teilchenextraktionsphase die Energie der von der Teilchenbeschleunigervorrichtung freigesetzten Teilchen zumindest zeitweise (III) und/oder zumindest teilweise zumindest quasi-kontinuierlich verändert wird. Die Teilchenbeschleunigervorrichtung ist derart ausgebildet und eingerichtet, dass sie zumindest zeitweise und/oder zumindest teilweise ein vorab beschriebenes Verfahren durchführen kann beziehungsweise durchführt. Die Teilchenbeschleunigervorrichtung weist dann die bereits vorab beschriebenen Eigenschaften und Vorteile in analoger Weise auf. Dabei ist es an sich beliebig, ob das Verfahren softwaremäßig und/oder hardwaremäßig implementiert ist. Üblicherweise ist es besonders vorteilhaft, wenn bestimmte Teile des Verfahrens softwaremäßig realisiert werden, während andere Teile des Verfahrens hardwaremäßig (beispielsweise durch geeignete Anpassung von Komponenten und/oder zusätzlichen Einbau von Komponenten) umgesetzt werden. Auch kann die Teilchenbeschleunigervorrichtung im Sinne der vorherigen Beschreibung des Verfahrens weitergebildet werden.

Die Teilchenbeschleunigervorrichtung weist zumindest eine Steuervorrichtung auf, welche zumindest zeitweise und/oder zumindest teilweise während einer Teilchenextraktionsphase ein Energie-Sollsignal ausgibt, verarbeitet und/oder empfängt. Dieses Energie-Sollsignal kann beispielsweise zumindest teilweise in Abhängigkeit von einer erwünschten Eindringtiefe in ein zu bearbeitendes Werkstück (zum Beispiel einen Patienten), zumindest teilweise in Abhängigkeit von der sich verändernden Lage eines sich bewegenden Werkstücks, beispielsweise eines sich an einem bewegenden Organ befindenden Tumors und/oder zumindest teilweise in Abhängigkeit von ermittelten Messwerten generiert werden. Die Steuervorrichtung kann dabei (teilweise) als gesonderte Vorrichtung ausgebildet sein und/oder (teilweise) softwaremäßig implementiert werden und gegebenenfalls auf einer bei Teilchenbeschleunigern üblicherweise ohnehin vorhandenen Computereinrichtung mit ausgeführt werden.

Bei der Teilchenbeschleunigervorrichtung ist weiterhin zumindest eine Steuervorrichtung vorgesehen, die zumindest eine Speichervorrichtung zur Speicherung von Geräteparametern in Abhängigkeit von einem Energie-Sollsignal aufweist. Es ist möglich, dass die Steuervorrichtung zumindest eine Speichervorrichtung, in der Geräteparameter in Abhängigkeit von einem Energie-Sollsignal gespeichert sind, aufweist und/oder dass zumindest eine Steuervorrichtung vorgesehen ist, die zumindest eine Recheneinheit zur Interpolation und/oder Extrapolation von in zumindest einer Speichervorrichtung gespeicherten Daten aufweist. Mit einer derart ausgebildeten Vorrichtung ist es insbesondere möglich, besonders schnell von einem Energie-Sollwert auf zu verwendende Geräte-Parameter zu schließen (beispielsweise auf elektrische Ströme, mit denen Magnete angesteuert werden; die Stärke und Frequenz von elektromagnetischen Wanderfeldern und dergleichen). Dadurch kann die Genauigkeit des Beaufschlagungsorts des von der Teilchenbeschleunigervorrichtung erzeugten Teilchenstrahls besonders hoch sein. Alternativ oder zusätzlich kann auch die Zeitdauer, mit der ein "Look-Ahead"-Algorithmus in die Zukunft "schauen" muss, reduziert werden, was ebenfalls die Genauigkeit der Energie und/oder Position erhöhen kann.

Es ist ebenfalls eine Steuervorrichtung zum Einbau in eine Teilchenbeschleunigervorrichtung, die einen Synchrotronring mit einzelnen Komponenten, die für eine langsame Extraktion angeordnet sind, aufweist, insbesondere zur Durchführung eines erfindungsgemäßen Verfahrens, vorgesehen, die zumindest eine Speichervorrichtung zur Speicherung von Geräteparametern in Abhängigkeit von einem Energie-Sollsignal aufweist und/oder zumindest eine Speichervorrichtung, in der Geräteparameter in Abhängigkeit von einem Energie-Sollsignal gespeichert sind, aufweist, wobei die Steuereinheit derart ausgebildet und eingerichtet ist, dass die Energie der von der Teilchenbeschleuniger¬vorrichtung (1) freigesetzten Teilchen (23) durch Veränderung der Energie der Teilchen (23) in der Teilchenbeschleunigervorrichtung (1) während der Teilchenextraktionsphase zumindest zeitweise (III) und/oder zumindest teilweise zumindest quasi-kontinuierlich verändert (27) wird. Die Steuervorrichtung kann zumindest eine Recheneinheit zur Interpolation und/oder Extrapolation von in zumindest einer Speichervorrichtung gespeicherten Daten aufweisen.

Im Folgenden wird die Erfindung anhand vorteilhafter Ausführungsbeispiele und unter Bezugnahme auf die beigefügte Zeichnung näher erläutert. Es zeigen:
- Fig. 1:: den Prinzipaufbau eines Synchrotron-Teilchenbeschleunigers;
- Fig. 2:: eine schematische Veranschaulichung eines bekannten Verfahrens zur kontinuierlichen Anpassung der Energie eines Teilchenstrahls;
- Fig. 3:: die Energieanpassung des Teilchenstrahls bei einer langsamen Strahlextraktion aus einem Synchrotron zur Bestrahlung eines sich bewegenden Zielvolumens;
- Fig. 4:: eine mögliche Ausführungsform für einen "Look-Ahead"-Algorithmus zur Vorab-Bestimmung einer Soll-Strahlenergie;
- Fig. 5:: ein Verfahren zur schnellen Extrapolation neuer Steuerungsdaten aus den jeweils vorangegangenen Werten;
- Fig. 6:: ein Verfahren zur Ermittlung von Steuerparametern aus einer look-up-Tabelle mithilfe eines Interpolationsverfahrens;
- Fig. 7:: der prinzipielle Grundaufbau einer Rückkopplungsschaltung zur Verwendung bei der Bestrahlung eines sich bewegenden Zielvolumens.

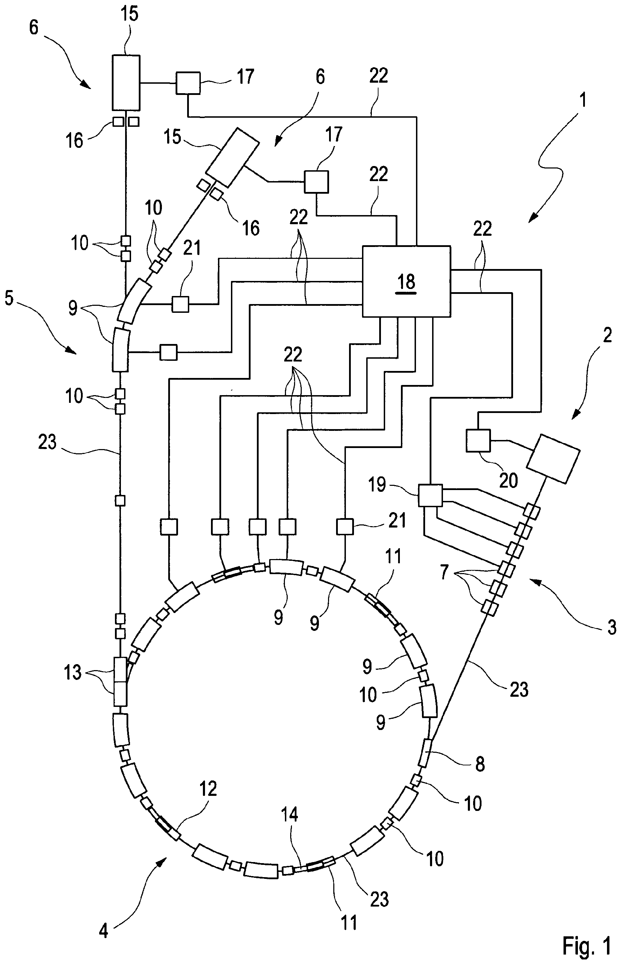

In Fig. 1 ist schematisch der Prinzipaufbau eines möglichen Ausführungsbeispiels einer Teilchenbeschleunigervorrichtung, einer Synchrotronanlage 1 dargestellt. Grundsätzlich kann man die Synchrotronanlage 1 in eine Mehrzahl von Untereinheiten aufteilen:
- in eine lonenquelle 2, in der die zu beschleunigenden Ionen erzeugt werden (einschließlich Ionisierung derselben);
- in eine Vorbeschleunigerstrecke 3, in der die in der lonenquelle 2 erzeugten Ionen zur Injektion in den Synchrotronring 4 vorbeschleunigt werden. Die Vorbeschleunigerstrecke 3 ist in der Regel als Linearbeschleuniger 3 (typischerweise als "Linac" für "LINear ACcelerator" bezeichnet); ausgebildet;
- in den eigentlichen Synchrotronring 4, in dem die Teilchen von einer niedrigen Anfangsgeschwindigkeit (vom Linearbeschleuniger 3 geliefert) im Laufe von typischerweise wenigen Sekunden auf eine gewünschte, hohe Endenergie beschleunigt werden;
- in die Strahltransportstrecke 5, in der die im Synchrotronring 4 auf eine hohe Energie beschleunigten Ionen zu dem oder den Behandlungsplätzen 6 transportiert werden;
- und schließlich in die eigentlichen Behandlungsplätze 6, einschließlich etwaiger dort erforderlicher Zusatzkomponenten.

Die Übergabe des Teilchenstrahls vom Linearbeschleuniger 3 in den Synchrotronring 4 erfolgt durch an sich bekannte Injektionsmittel 8. Dementsprechend erfolgt die Extraktion des Teilchenstrahls aus dem Synchrotronring 4 in die Strahltransportstrecke 5 durch an sich bekannte Extraktionsmittel 12, 13.

Auch die lonenquelle 2 ist an sich bekannt und besteht beispielsweise aus einer Verdampfungsvorrichtung sowie (soweit erforderlich) geeigneten lonisierungsmitteln, um die verdampften Atome zu ionisieren und damit bebeschleunigbar zu machen. Falls die "Atome" beispielsweise durch eine Gasentladung und/oder durch Beschuss mit einer Elektronenkanone freigesetzt werden, liegen diese üblicherweise ohnehin in Form von Ionen vor.

Der Linearbeschleuniger 3 besteht üblicherweise aus dem vakuumbeaufschlagten Beschleunigerrohr (ein Vakuumrohr) sowie einer Mehrzahl von Strahlbeschleunigungsmitteln 7, die beispielsweise mit einer hochfrequenten Wechselspannung beaufschlagt werden. Das Strahlinjektionsmittel 8 liegt beispielsweise in Form eines schnell schaltbaren Dipolmagneten vor.

Der Synchrotronring 4 weist in an sich bekannter Weise eine größere Anzahl jeweils paarweise angeordneter Dipolmagnete 9 auf, die den Teilchenstrahl auf eine umlaufende Bahn zwingen. Zwischen jeweils zwei Dipolmagneten 9 ist jeweils ein Quadrupolmagnet 10 angeordnet. Die Quadrupolmagnete 10 bewirken jeweils eine Fokussierung des lonenstrahls 23 im einer ersten Richtung (beispielsweise in der senkrechten Richtung) wohingegen sie eine Defokussierung in einer dazu senkrecht stehenden, zweiten Richtung (beispielsweise in der waagrechten Richtung) bewirken. Bei einer Hintereinanderschaltung von mehreren jeweils zueinander "verdrehten" Quadrupolmagneten 10 kommt es jedoch im Endergebnis zu einer Fokussierung des lonenstrahls 23 in beiden Richtungen. Weiterhin sind entlang der Teilchenbahn Strahlbeschleunigungsmittel 11 angeordnet, die eine Beschleunigung (Energieerhöhung) des umlaufenden lonenstrahls 23 (der üblicherweise in Form von mehreren Teilchenpaketen, so genannten Bunches, vorliegt) bewirken. Zur Extraktion des im Synchrotronring 4 umlaufenden lonenstrahls 23 dienen im vorliegend dargestellten Ausführungsbeispiel insgesamt sechs Sextupolmagnete, ein elektrostatisches Extraktionsseptum 12, zwei Septummagnete 13 sowie eine hochfrequente Rauschanregung 14 (beispielsweise durch einen so genannten Exciter) des im Synchrotronring 4 umlaufenden lonenstrahls 23.

Über die Stahltransportstrecke 5 wird der aus dem Synchrotronring 4 extrahierte lonenstrahl 23 über zum Teil schaltbare Dipolmagnete 9 dem jeweils aktiven Behandlungsplatz 6 zugeführt. Auch entlang der Strahltransportstrecke 5 sind vorzugsweise weitere Quadrupolmagnete 10 vorgesehen, die der Nach-Fokussierung des lonenstrahls 23 dienen. Im Bereich der Behandlungsplätze 6 ist vor der eigentlichen Patientenliege 15 jeweils eine Rasterscanneranordnung unter Verwendung von Scannermagneten 16 vorgesehen. Zusätzlich können noch vorliegend nicht dargestellte Schnellabschaltmittel vorgesehen werden, um den Teilchenstrahl im Falle eines Fehlers innerhalb kürzester Zeit (typischerweise innerhalb von weniger als 1 Millisekunde) schnell abschalten zu können, um Verletzungen des Patienten zu vermeiden. Die Schnellabschaltmittel werden typischerweise im Bereich des Synchrotronrings 4 und/oder der Strahltransportstrecke 5 vorgesehen.

Die gesamte Synchrotronanlage 1 weist eine größere Anzahl an elektronischen Steuereinheiten 17-21 auf, da die Synchrotronanlage 1 aus "Performance"-Gründen dezentral ausgelegt ist. So weisen die einzelnen Behandlungsplätze 6 jeweils einen eigenen Behandlungsplatzrechner 17 auf, in die jeweils der passende Behandlungsplan für den aktuell zu behandelnden Patienten eingespeist wird. Der Behandlungsplatzrechner 17 verarbeitet dabei auch Messwerte, die beispielsweise von Strahlpositionsdetektoren (beispielsweise Vieldrahtkammern) geliefert werden. Auch die lonenquelle 2 und der Linearbeschleuniger 3 weisen einen dezidierten lonenquellenrechner 20 beziehungsweise einen dezidierten Linearbeschleuniger-Rechner 19 (Linac-Rechner) auf. Darüber hinaus sind auch die einzelnen Komponenten des Synchrotronrings 4 sowie der Strahltransportstrecke 5 (insbesondere Dipolmagnete 9, Quadrupolmagnete 10, Sextupolmagnete, Strahlbeschleunigungsmittel 11, elektrostatisches Extraktionsseptum 12, Septummagnete 13, Rauschgenerator 14 sowie Strahlinjektionsmittel 8) jeweils mit einem Komponentenrechner 21 versehen (aus Übersichtlichkeitsgründen sind die Komponentenrechner 21 nur teilweise dargestellt). Die Komponentenrechner 21 können nicht nur als klassische Computer, sondern insbesondere auch als Einplatinencomputer oder dergleichen ausgeführt sein. Sämtliche individuellen Rechner 17, 19-21 (also Behandlungsplatzrechner 17, Linearbeschleuniger-Rechner 19, Ionenquellenrechner 20 und Komponentenrechner 21; gegebenenfalls auch weitere, in Fig. 1 nicht dargestellte Einzelrechner) sind über bidirektionale Datenleitungen 22 mit einem Zentralrechner 18 verbunden, der die zentrale Einheit für die Gesamtsteuerung der Synchrotronanlage 1 darstellt. Die bidirektionalen Datenleitungen 22 können im Übrigen nicht nur als Einzelleitungen, sondern auch als (teilweise) zusammengefasste Datenbusleitungen (wie beispielsweise Ethernet-Datenbusse, Token-RingSysteme, Glasfaserkabel und dergleichen) ausgeführt sein, an die jeweils mehrere Komponenten angeschlossen sind. Die Gruppierung der einzelnen Rechner sollte dabei derart erfolgen, dass die jeweiligen Datenbusse eine ausreichende Datenübertragungskapazität aufweisen (vorzugsweise noch mit einer bestimmten Kapazitätsreserve).

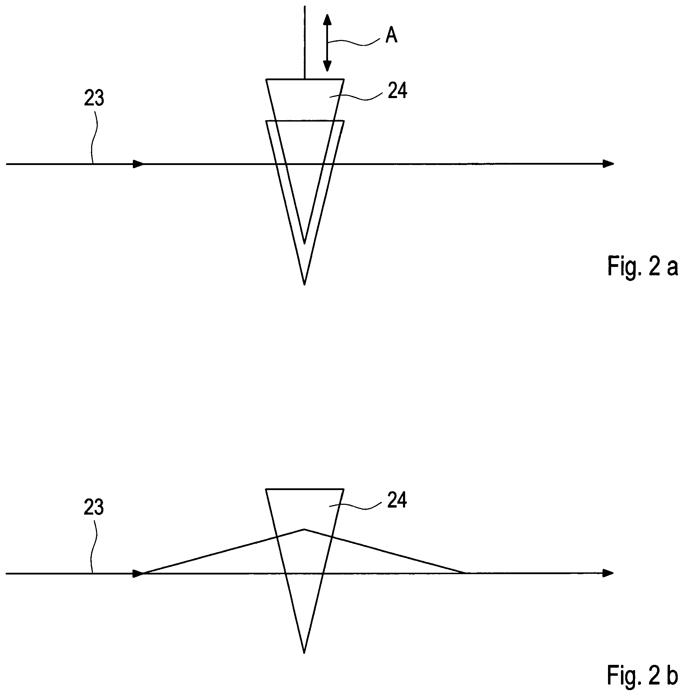

In Fig. 2, Figuren 2a und 2b ist aus Veranschaulichungsgründen ein bekanntes Verfahren zur kontinuierlichen Veränderung der Energie eines lonenstrahls 23 dargestellt. Der lonenstrahl 23 wird dabei durch ein energieabsorbierendes Material 24 geschickt, in dem er einen Energieverlust erfährt. Der Energieverlust des lonenstrahls 23 ist dabei umso größer, je länger die Strecke ist, die er in dem energieabsorbierenden Material 24 zurücklegen muss. Bewegt man nun das in Fig. 2a dargestellte, vorliegend keilförmig ausgeformte energieabsorbierende Material 24 (in Fig. 2a durch einen Doppelpfeil A angedeutet), so kann man die Wegstrecke, die der lonenstrahl 23 durch das energieabsorbierende Material 24 hindurch läuft, variieren (in Fig. 2a sind aus Veranschaulichungsgründen zwei unterschiedliche Positionen des energieabsorbierenden Materials 24 eingezeichnet). Durch kontinuierliche Repositionierung des energieabsorbierenden Materials 24 kann eine kontinuierliche Energievariation realisiert werden, auch wenn die Energie des einfallenden lonenstrahls 23 konstant ist. Möglich ist es auch, den lonenstrahl 23 durch laterale Ablenkung (siehe Fig. 2b) unterschiedliche Wege durch das keilförmig ausgebildete energieabsorbierende Material 24 zurücklegen zu lassen.

Beide Verfahren sind passive Methoden zur Energieeinstellung. Die gravierenden Nachteile dieser Verfahren bestehen insbesondere darin, dass es zu einer Aufstreuung des lonenstrahls sowie zu einer Verbreiterung der Energieverteilung im lonenstrahl durch Energieaufstreuung (sogenanntes energy straggling) kommt. Diese Nachteile können oftmals nur teilweise durch geeignete Blenden-Analysiersysteme kompensiert werden. Dabei kommt es jedoch in der Regel zu hohen Strahlverlusten, zum Teil im Bereich von über 90 %, sowie zu einer vermeidbaren radioaktiven Kontamination von Komponenten (aufgrund des dann in der Regel erforderlichen Strahldumpings), was unerwünscht ist. Darüber hinaus stellen die Blenden-Analysiersysteme auch einen zusätzlichen Aufwand dar.

Es wird somit vorgeschlagen, die Energie der aus dem Synchrotronring 4 extrahierten Teilchen während der Extraktionsphase (quasi-) kontinuierlich zu verändern. Im vorliegend dargestellten Ausführungsbeispiel der Synchrotronanlage 1 wird dabei eine langsame Extraktion verwendet, die typischerweise im Bereich von 10 Sekunden andauert. Eine langsame Extraktion ist vorteilhaft, um die in jedem einzelnen Voxel deponierte Energiedosis genauer einhalten zu können und insbesondere eine Überdosierung zu vermeiden.

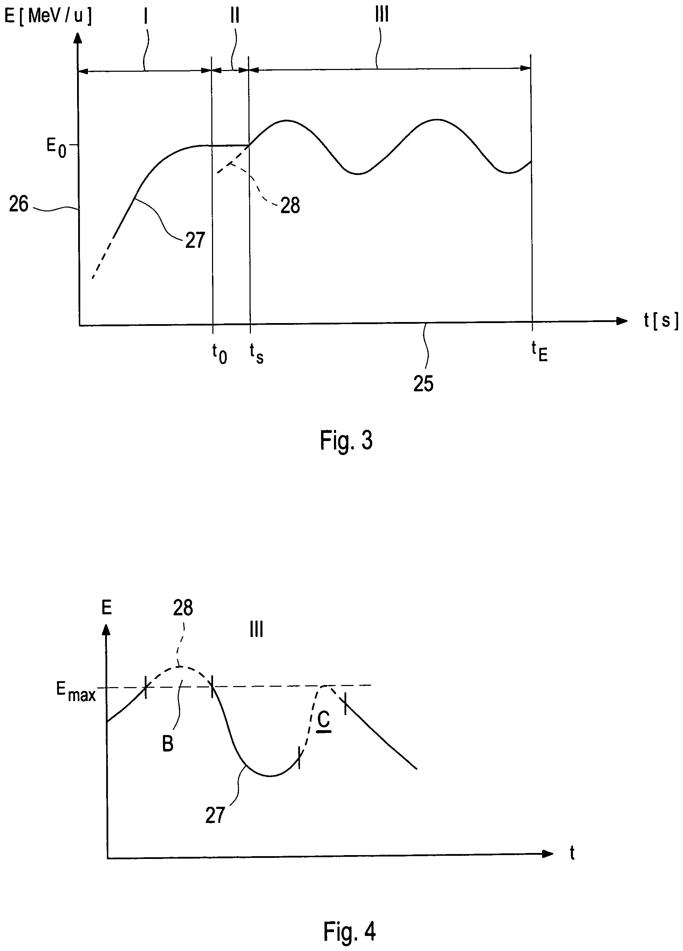

In Fig. 3 ist die Energie E eines im Synchrotronring 4 befindlichen Teilchens (längs der Ordinate 26 aufgetragen) über die Zeit t (längs der Abszisse 25 aufgetragen) dargestellt. Im ersten Zeitintervall I werden die Teilchen von einer niedrigen Energie (welche von der Vorbeschleunigung durch den Linearbeschleuniger 3 herrührt) innerhalb einer kurzen Zeitspanne von typischerweise etwa ein bis zwei Sekunden auf einen Energieinitialwert E₀ beschleunigt. Da zu diesem Zeitpunkt die Extraktionsmittel (Sextupolmagnete, Extraktionsseptum 12, Septummagnete 13, Rauschanregung 14) ausgeschaltet sind, ist der lonenstrahl 23 relativ stabil. Aufgrund dieser Stabilität können gewisse Schwankungen der Soll-Parameter des Synchrotronrings 4 (beispielsweise Schwankungen der Magnetfeldstärken der Dipolmagneten 9 und/oder der Quadrupolmagneten 10 sowie der elektrischen Felder der Strahlbeschleunigungsmittel 11) relativ groß sein, ohne dass es zu einer übermäßigen Verschlechterung des umlaufenden lonenstrahls 23 beziehungsweise gar zu einem Verlust des lonenstrahls 23 kommt. Zum Ende der initialen Beschleunigungsphase hin (Endbereich von Zeitintervall I) schmiegt sich die lonenenergie 27 an den initialen Energiewert E₀ an. Am Ende der initialen Beschleunigungsphase I (zum Zeitpunkt t₀) liegt der Energieinitialwert E₀ im Synchrotronring 4 vor. Dieser Wert ist jedoch nicht notwendigerweise geeignet, um unverzüglich mit der Strahlextraktion beginnen zu können.

Beispielsweise soll beim in Fig. 3 dargestellten Beispiel ein Tumor behandelt werden, welcher in der Lunge eines Patienten sitzt. Dieser Tumor bewegt sich mit dem Atemrhythmus des Patienten, was durch die Atemlinie 28 dargestellt wird. Die Atemlinie 28 zeigt die Energie an, die erforderlich ist, damit sich der Bragg-Peak des lonenstrahls 23 im erwünschten Bestrahlungsvoxel (der üblicherweise innerhalb des Tumors liegt) befindet.

Wie man aus Fig. 3 unschwer erkennen kann, liegt zum Zeitpunkt t₀ eine Diskrepanz zwischen der Ionenenergie 27 und der Atemlinie 28 vor. Aufgrund der Periodizität der Atembewegung ist jedoch absehbar, dass beide Energien in naher Zukunft (nämlich zum Zeitpunkt tₛ) übereinstimmen werden. Um unnötige Energieanpassungen (um besonders "schnell" mit der Extraktion beginnen zu können; eine sprungartige Energieanpassung ist ohnehin nicht möglich) zu vermeiden, wird daher ein Warteintervall II initiiert, in dem einfach so lange gewartet wird, bis die initiale Energie E₀ mit der (zum entsprechenden Zeitpunkt erforderlichen) Soll-Energie übereinstimmt.

Zu diesem Zeitpunkt tₛ (Ionenenergie 27 und Atemlinie 28 treffen erstmals zusammen) wird mit der Extraktionsphase III, also mit der (langsamen) Strahlextraktion und damit mit der Bestrahlung des Patienten begonnen. Damit sich das zu bestrahlende Voxel nicht aus dem aktiven Bestrahlungsbereich heraus bewegt, wird nunmehr die Ionenenergie 27 parallel zur Atemlinie 28 nachgeführt. Diese Energienachführung ist ebenfalls aus Fig. 3 ersichtlich. Nach Ablauf einer bestimmten Zeit ist der im Synchrotronring 4 verbliebene lonenstrahl 23 erschöpft, so dass keine weiteren Ionen mehr aus dem Synchrotronring 4 entnommen werden können. Zu diesem Zeitpunkt endet die Strahlextraktionsphase III (t_{E}). Anschließend erfolgt in aller Regel eine neue Injektionsphase (nicht dargestellt), eine erneute Beschleunigungsphase I, gegebenenfalls eine (erneute) Wartephase II sowie eine anschließende Strahlextraktionsphase III.

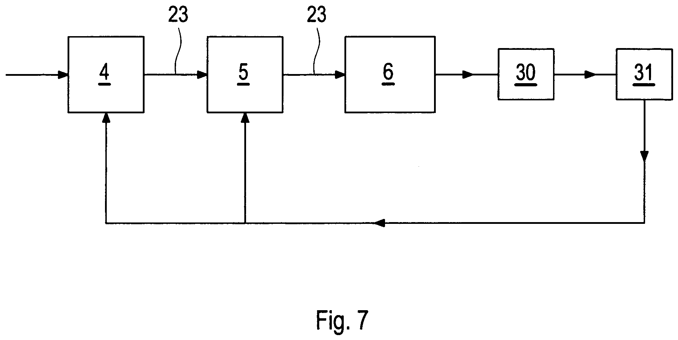

Da die erforderlichen Energiekorrekturen während der Strahlextraktionsphase III deutlich kleiner sind als während der initialen Beschleunigungsphase I, sind die Änderungsraten der Energie pro Zeiteinheit *dE*/*dt* (Rampensteilheit der Ionenenergiekurve 27 im Intervall III) ausreichend klein, sodass die einzelnen Komponenten des Synchrotronrings 4 während einer laufenden Extraktion ausreichend genau sowie ausreichend schnell nachgesteuert werden können. Um die Genauigkeit der einzelnen Komponenten zu erhöhen, können dabei zusätzliche Maßnahmen ergriffen werden. Eine besonders vorteilhafte Maßnahme besteht beispielsweise darin, die Stärke der von den Magneten (insbesondere der von den Dipolmagneten 9 und der von den Quadrupolmagneten 10) erzeugten Magnetfelder aktiv zu messen und die aktuell gemessenen Magnetfeldstärken Bᵢₛₜ mit einem Soll-Magnetfeld Bₛₒₗₗ zu vergleichen. Basierend auf diesem Vergleichsergebnis können dann die elektrischen Ströme, mit denen die Magnete 9, 10 angesteuert werden, geeignet nachgeführt werden (vergleiche auch Fig. 7).

In Fig. 4 ist dargestellt, wie die Nachführung der Ionenenergie 27 gegebenenfalls während bestimmter Zeitintervalle ausgesetzt werden kann. So kann es beispielsweise vorkommen, dass während eines bestimmten Zeitintervalls B während der Extraktionsphase III ein vom Synchrotronring 4 erreichbarer Maximal-Energiewert Eₘₐₓ überschritten wird, was in Fig. 4 im Intervall B durch eine gestrichelte Linie angedeutet ist (korrespondierend dazu kann auch ein Energie-Minimalwert Eₘᵢₙ des Synchrotronrings 4 unterschritten werden). In diesem Zeitintervall B kann die Ionenenergie 27 technisch bedingt nicht der Atemlinie 28 folgen. Daher wird in diesem Zeitintervall B die Strahlextraktion kurzfristig unterbrochen und anschließend wieder fortgesetzt. In der Realität kann dies beispielsweise auftreten, wenn der Patient infolge von Nervosität besonders tief einatmet.

Ebenso ist es möglich, dass eine bestimmte maximale Energieveränderung pro Zeiteinheit *dE*/*dt* kurzzeitig überschritten wird (vergleiche Zeitintervall C in Fig. 4). Hier ändert sich die Atemlinie 28 schneller als die Ionenenergie 27 dieser folgen kann. Auch in diesem Fall wird das Überschreiten der Geräteparameter des Synchrotronrings 4 von einer Verifikationseinrichtung (welche beispielsweise softwaremäßig im Zentralrechner 18 implementiert sein kann) festgestellt und die Teilchenextraktion wird kurzfristig ausgesetzt. Dies ist ebenfalls in Fig. 4 angedeutet . Dies kann in der Realität beispielsweise durch einen Hustenreiz eines Patienten verursacht werden.

Vorzugsweise sollte der Synchrotronring 4 derart ausgelegt werden, dass Ereignisse, die zu einer kurzzeitigen Unterbrechung der lonenstrahlextraktion führen, möglichst einen nur relativ geringen Zeitanteil ausmachen, um Behandlungszeit einsparen zu können.

Um die Bestrahlungsgenauigkeit weiter zu erhöhen ist bei der Ansteuerung des vorliegend dargestellten Ausführungsbeispiels einer Synchrotronanlage 1 ein "Look-Ahead"-Algorithmus implementiert, mit dem die zu erwartende Position des zu bestrahlenden Gewebes (beispielsweise Rasterpunktes oder Voxels) in der unmittelbar bevorstehenden Zukunft extrapoliert wird. Dies ist insbesondere bei sich wiederholenden Bewegungsabläufen des Zielvolumens möglich.

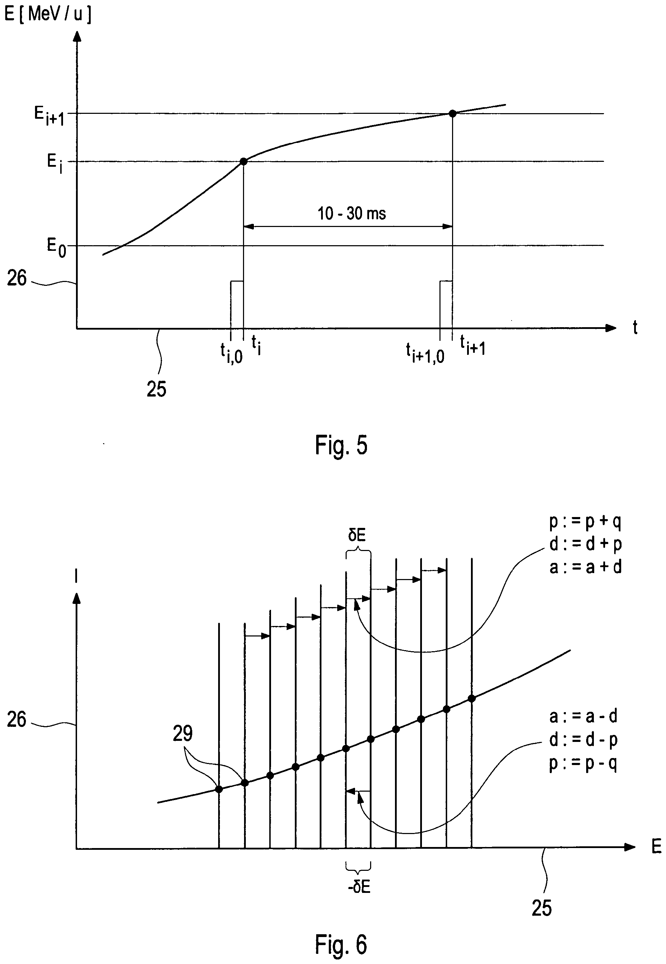

In Fig. 5 ist das hierzu verwendete Grundprinzip exemplarisch dargestellt. Die Extrapolation ist insbesondere bei für medizinische Zwecke benutzten Synchrotronanlagen 1 möglich, weil bei den sich bewegenden Zielvolumen oftmals periodische Bewegungsabläufe gegeben sind. Beispielsweise folgt die Atembewegung einer Person in aller Regel einem für die betreffende Person im Wesentlichen wiederkehrenden Bewegungsablauf (obgleich sich der Bewegungsablauf von Person zu Person unterscheiden kann). Hierzu wird jeweils zu bestimmten Zeitpunkten tᵢ, tᵢ₊₁ ... die Bewegung des zu bestrahlenden Zielvolumens für den jeweils nächsten Zeitschritt tᵢ₊₁, tᵢ₊₂ ... extrapoliert. Der erwartete Ort des zu bestrahlenden Voxels ist dabei in Fig. 5 bereits in Energiewerte Eᵢ, Eᵢ₊₁ ... (längs der Ordinate 26 aufgetragen) umgerechnet. Beim Beginn des jeweils nächsten Zeitintervalls tᵢ, tᵢ₊₁ ... wird dabei jeweils eine neue Teilchenenergie Eᵢ, Eᵢ₊₁ ... eingestellt.

Das in Fig. 5 längs der Abszisse 25 schematisch angedeutete Zeitintervall von t_{i,0} bis tᵢ beziehungsweise t_{i+1,0} bis tᵢ₊₁ bezeichnet dabei den zeitlichen Vorlauf der erforderlich ist, um die jeweils berechneten Energiewerte Eᵢ, Eᵢ₊₁ ... über die Datenleitungen 22 vom Zentralrechner 18 an die entsprechenden Einzelrechner 17, 19, 20, 21 zu übertragen. Der zeitliche Abstand zwischen zwei Einzelberechnungsschritten tᵢ, tᵢ₊₁ ... beträgt typischerweise 20 Millisekunden, kann aber ohne Weiteres auch zwischen 10 und 30 Millisekunden betragen (je nach konkretem Anwendungsgebiet auch mehr oder weniger).

In Fig. 6 ist dargestellt, wie beispielsweise bei einem Komponentenrechner 21, der der Ansteuerung eines Dipolmagneten 9 beziehungsweise eines Quadrupolmagneten 10 dient, die angeforderte Sollenergie E (längs der Abszisse 25 aufgetragen) in einen Geräteparameter (vorliegend den Magnetstrom I) umgerechnet wird. Die Abhängigkeit zwischen E und I ist dabei nichtlinear. Im Speicher des Komponentenrechners 21 ist dazu eine Mehrzahl an Datenpunkten 29 abgespeichert. Je nach konkretem Einsatzerfordernis kann die genaue Anzahl der Datenpunkte 29 größer oder kleiner gewählt werden. Nachteilig bei einer großen Anzahl an Datenpunkten 29 ist, dass der Speicher entsprechend groß dimensioniert sein muss und eine entsprechend große Anzahl an Datenpunkten 29 ausgemessen werden muss. Um den Speicherbedarf nicht übermäßig ansteigen zu lassen und um die Kalibrierung der Synchrotronanlage 1 nicht zu zeitaufwändig werden zu lassen, werden üblicherweise nicht übermäßig viele Datenpunkte 29 (typischerweise im Bereich von 100 Datenpunkten) in dem Komponentenrechner 21 gespeichert. Wird ein Zwischenenergiewert abgefragt, der kleiner als das durch die Datenpunkte 29 vorgegebene Energieraster *δE* ist, so wird der entsprechende Zwischenwert des Magnetstroms I entsprechend interpoliert. Bewährt hat sich hierbei insbesondere eine kubische Interpolation, weil sich gezeigt hat, dass hierdurch instabile Oszillationen durch die Interpolation vermieden werden können.

Die aus der "Look-Up"-Tabelle (beziehungsweise aus den Interpolationen) gewonnenen Stromwerte I werden dabei vorzugsweise zumindest zeitweise durch eine Feedback-Regelschleife nachgesteuert. Dies gilt insbesondere während des Warteintervalls II beziehungsweise während des Extraktionsintervalls III (vergleiche Fig. 3). Zu diesen Zeitpunkten wird das tatsächlich vom aktuell anliegenden Magnetstrom I_{M} erzeugte magnetische Feld Bᵢₛₜ mit dem Soll-Magnetfeld Bₛₒₗₗ (welches beispielsweise aus einer Tabelle unter Verwendung von Interpolationsverfahren als eine Funktion Bₛₒₗₗ(I) ausgedrückt werden kann) verglichen. Liegt eine Abweichung vor, so wird der Magnetstrom I_{M}, wie er aus der in Fig. 6 dargestellten Look-Up-Tabelle entnommen werden kann, entsprechend nachgesteuert. Dadurch ist eine höhere Genauigkeit des tatsächlich erzeugten Magnetfelds Bᵢₛₜ möglich, die insbesondere zu Zeiten einer Teilchenstrahlextraktion III von Vorteil ist. Zu diesen Zeitpunkten ändert sich typischerweise die Magnetfeldstärke Bₛₒₗₗ nicht übermäßig schnell mit der Zeit, so dass gewisse zeitliche Regelkreis-Verzögerungen nicht besonders problematisch sind. Mithilfe des vorgeschlagenen Magnetfeld-Feedback-Algorithmus ist es jedenfalls insbesondere auch möglich, auf besonders einfache Weise magnetische Remanenzen und dergleichen berücksichtigen zu können.

Ein derartiger Feedback-Algorithmus benötigt jedoch eine gewisse Regelzeit, so dass dessen Anwendung bei sich besonders schnell ändernden Magnetfeldern nicht nur wenig hilfreich sein kann, sondern ganz im Gegenteil sogar von Nachteil sein kann. Aus diesem Grund erfolgt während des initialen Beschleunigungszyklus I keine derartige Rückkopplung. Zu diesem Zeitpunkt wird ausschließlich die in Fig. 6 veranschaulichte Look-Up-Tabelle angewendet. Wie bereits erwähnt, ist der lonenstrahl 23 während der initialen Beschleunigungsphase I jedoch relativ stabil, so dass die dadurch induzierten Magnetfeldstärkefehler keinen übermäßig negativen Einfluss auf den lonenstrahl 23 haben.

In Fig. 7 ist schließlich noch schematisch der Regelkreis dargestellt. Mittels des Regelkreises kann zumindest teilweise das Verfahren zum Betrieb der Teilchenbeschleuniger-Vorrichtung durchgeführt werden. Der Regelkreis weist den prinzipiellen Aufbau einer Rückkopplungsschaltung auf, die bei dem Betreiben einer Teilchenbeschleunigungsvorrichtung bei der Bestrahlung eines Werkstücks und/oder Gegenstands, beispielsweise eines Patienten verwendet wird. Der Regelkreis realisiert eine Energienachführung in Abhängigkeit von der Bewegung des zu bestrahlenden Zielvolumens. Der Patient am Behandlungsplatz 6 wird beispielsweise während der fortlaufenden Behandlung, also Bestrahlung mit dem Teilchenstrahl, durch eine Einrichtung 30 untersucht, die Daten über die aktuelle Position des zu bestrahlenden Gewebes - und damit des zu bestrahlenden Rasterpunktes oder Voxels - liefert. Diese Daten werden in einer Steuereinheit 31 in eine Energievorgabe umgesetzt. Die Steuereinheit 31 kann beispielsweise Teil eines Behandlungsplatzrechners 17 oder des Zentralrechners 18 sein. Die Energievorgabe umfasst hierbei die zur Bestrahlung des jeweiligen Rasterpunktes oder Voxels geeignete Energie des Teilchenstrahls. Dadurch ist eine angepasste Position des Bragg-Peaks sichergestellt. Die Energievorgaben werden insbesondere an die Komponenten des Synchrotronrings 4 sowie die Komponenten der Strahltransportstrecke 5 übermittelt. Hierdurch wird ein lonenstrahl 23 mit geeigneter Energie am Behandlungsplatz 6 bereitgestellt.

Weitere Informationen zur vorliegenden Erfindung können der deutschen Patentanmeldung mit dem amtlichen Aktenzeichen DE 10 2008 047 197 A1 mit Anmeldetag vom 29. September 2008 entnommen werden.

Die Einrichtung 30 kann beispielsweise einen Computertomograph umfassen. Die Einrichtung 30 kann aber auch jede Einrichtung sein, die eine Bewegung des zu bestrahlenden Zielvolumens im Patienten zu detektieren vermag. Gegebenenfalls kann die detektierte Bewegung in eine aktuelle Position des zu bestrahlenden Voxels oder Rasterpunkts transformiert oder umgerechnet werden. Derartige Einrichtungen zur Detektion der Bewegung, beispielsweise der durch Atmung verursachten Bewegung eines Zielvolumens sind an sich bekannt.

**BEZUGSZEICHENLISTE**

| | |
|---|---|
| 1 Synchrotron | 19 Linearbeschleuniger-Rechner |
| 2 Ionenquelle | 20 Ionenquellenrechner |
| 3 Linearbeschleuniger | 21 Komponentenrechner |
| 4 Synchrotronring | 22 bidirektionale Datenleitung |
| 5 Strahltransportstrecke | 23 Ionenstrahl |
| 6 Behandlungsplatz | 24 energieabsorbierendes Material |
| 7 Strahlbeschleunigungsmittel | 25 Abszisse |
| 8 Strahlinjektionsmittel | 26 Ordinate |
| 9 Dipolmagnet | 27 Ionenenergie |
| 10 Quattropolmagnet | 28 Atemlinie |
| 11 Strahlbeschleunigungsmittel | 29 Datenpunkt |
| 12 elektrostatisches Extraktionsseptum | 30 Einrichtung zur Detektion der Bewegung eines Zielvolumens, |
| 13 Septummagnet | beispielsweise umfassend einen |
| 14 Rauschanregung | Computertomograph |
| 15 Patientenliege | 31 Steuereinheit |
| 16 Rasterscannermagnet | I Initiale Beschleunigung |
| 17 Behandlungsplatzrechner | II Warteintervall |
| 18 Zentralrechner | III Extraktionsintervall |

## Patentansprüche

1. Verfahren zum Betrieb einer Teilchenbeschleunigervorrichtung (1) nach Anspruch 8, die einen Synchrotronring mit einzelnen Komponenten, die für eine langsame Extraktion angeordnet sind; aufweist, **dadurch gekennzeichnet, dass** die Energie der von der Teilchenbeschleunigervorrichtung (1) freigesetzten Teilchen (23) während der Teilchenextraktionsphase durch Veränderung (27) der Energie der Teilchen (23) in der Teilchenbeschleunigervorrichtung (1) zumindest zeitweise (III) und/oder zumindest teilweise zumindest quasi-kontinuierlich verändert (27) wird.

2. Verfahren nach Anspruch 1, **dadurch gekennzeichnet, dass** es sich bei der Teilchenbeschleunigervorrichtung (1) um eine Teilchenbeschleunigervorrichtung für Hadronen, insbesondere für Protonen, für Pionen, für Ionen und/oder für Schwerionen handelt.

3. Verfahren nach einem der vorangehenden Ansprüche, **dadurch gekennzeichnet, dass** die Teilchen (23) zumindest zeitweise und/oder zumindest teilweise in einem ersten Schritt (I) auf einen Energie-Initialwert (E₀) beschleunigt werden und in einem zweiten Schritt (III) die Teilchen auf einen Energie-Sollwert (Eᵢ) beschleunigt und/oder verzögert werden.

4. Verfahren nach einem der vorangehenden Ansprüche, **dadurch gekennzeichnet, dass** die Energie der von der Teilchenbeschleunigervorrichtung (1) freigesetzten Teilchen (23) zumindest zeitweise und/oder zumindest teilweise in Abhängigkeit von einem Sollwert (Eᵢ) und/oder in Abhängigkeit von einem Messwert verändert wird, bevorzugt in Abhängigkeit von einer erforderlichen Eindringtiefe in ein zu bestrahlendes Werkstück und/oder in Abhängigkeit von der Lage eines zu bestrahlenden, insbesondere sich bewegenden Zielvolumens eines zu bestrahlenden Werkstücks.

5. Verfahren nach einem der vorangehenden Ansprüche, insbesondere nach Anspruch 4, **dadurch gekennzeichnet, dass** die Lage und/oder die Bewegung eines zu bestrahlenden, insbesondere sich bewegenden Zielvolumens mit einem Extrapolations-Algorithmus vorab bestimmt wird.

6. Verfahren nach einem der vorangehenden Ansprüche, **gekennzeichnet durch** zumindest ein Verifikationsmittel, welches überprüft, ob eine angeforderte Teilchenenergie und/oder eine angeforderte Veränderungsrate der Teilchenenergie noch innerhalb der Betriebsparameter der Teilchenbeschleunigervorrichtung (1, 4) liegt.

7. Verfahren nach einem der vorangehenden Ansprüche, insbesondere nach einem der Ansprüche 4 bis 6, **dadurch gekennzeichnet, dass** insbesondere im Bereich eines Zielvolumens zumindest zeitweise und/oder zumindest teilweise eine laterale Veränderung des von der Teilchenbeschleunigervorrichtung (1) erzeugten Teilchenstrahls (23) erfolgt und/oder ein eindimensionales, zweidimensionales oder dreidimensionales Scanningverfahren (16) durchgeführt wird.

8. Teilchenbeschleunigervorrichtung (1) zur Erzeugung beschleunigter Teilchen (23), umfassend
einen Synchrotronring mit einzelnen Komponenten, die für eine langsame Extraktion angeordnet sind, und
eine Steuervorrichtung (17, 18, 19, 20, 21, 31), um zumindest zeitweise und/oder zumindest teilweise während einer Teilchenextraktionsphase (III) ein Energie-Sollsignal auszugeben, zu verarbeiten und/oder zu empfangen,
wobei die Steuervorrichtung (17, 18, 19, 20, 21, 31) eine Speichervorrichtung zur Speicherung von Geräteparametern in Abhängigkeit des Energie-Sollsignals aufweist,
**dadurch gekennzeichnet, dass** die Steuervorrichtung zur Steuerung der einzelnen Komponenten so eingerichtet ist, dass durch Veränderung (27) der Energie der Teilchen (23) in der Teilchenbeschleunigervorrichtung (1) während der Teilchenextraktionsphase die Energie der von der Teilchenbeschleunigervorrichtung (1) freigesetzten Teilchen (23) zumindest zeitweise (III) und/oder zumindest teilweise zumindest quasi-kontinuierlich verändert (27) wird.

9. Teilchenbeschleunigervorrichtung (1) nach Anspruch 8, **gekennzeichnet durch** zumindest eine Steuervorrichtung (17, 18, 19, 20, 21, 31), die zumindest eine Speichervorrichtung, in der Geräteparameter in Abhängigkeit von einem Energie-Sollsignal gespeichert sind, aufweist und/oder welche zumindest eine Recheneinheit (17, 18, 19, 20, 21, 31) zur Interpolation und/oder Extrapolation von in zumindest einer Speichervorrichtung gespeicherten Daten aufweist.

10. Steuervorrichtung zum Einbau in eine Teilchenbeschleunigervorrichtung (1), die einen Synchrotronring mit einzelnen Komponenten, die für eine langsame Extraktion angeordnet sind, aufweist, wobei die Steuervorrichtung zumindest eine Speichervorrichtung zur Speicherung von Geräteparametern in Abhängigkeit von einem Energie-Sollsignal aufweist und/oder zumindest eine Speichervorrichtung, in der Geräteparameter in Abhängigkeit von einem Energie-Sollsignal gespeichert sind, aufweist,
**dadurch gekennzeichnet, dass** die Steuereinheit derart ausgebildet und eingerichtet ist, dass die Energie der von der Teilchenbeschleunigervorrichtung (1) freigesetzten Teilchen (23) durch Veränderung der Energie der Teilchen (23) in der Teilchenbeschleunigervorrichtung (1) während der Teilchenextraktionsphase zumindest zeitweise (III) und/oder zumindest teilweise zumindest quasi-kontinuierlich verändert (27) wird.

11. Steuervorrichtung nach Anspruch 10, **dadurch gekennzeichnet, dass** die Steuervorrichtung zumindest eine Recheneinheit (17, 18, 19, 20, 21, 31) zur Interpolation und/oder Extrapolation von in der Speichervorrichtung gespeicherten Daten aufweist.

## Claims

1. Method for operating a particle accelerator device (1) according to claim 8, having a synchrotron with individual components arranged for a slow extraction, **characterized in that** the energy of the particles (23) released by the particle accelerator device (1) is changed (27) at least quasi-continuously at least at times (III) and/or at least in part during the particle extraction phase by changing (27) the energy of the particles (23) in the particle accelerator device (1).

2. Method according to claim 1, **characterized in that** the particle accelerator device (1) is a particle accelerator device for hadrons, in particular for protons, pions, ions and/or heavy ions.

3. Method according to one of the preceding claims, **characterized in that** the particles (23) are accelerated at least at times and/or at least in part to an initial energy value (E₀) in a first step (I), and the particles are accelerated and/or decelerated to a set energy value (Eᵢ) in a second step (III).

4. Method according to one of the preceding claims, **characterized in that** the energy of the particles (23) released by the particle accelerator device (1) is changed at least at times and/or at least in part depending on a set value (Eᵢ) and/or depending on a measured value, preferably depending on a required penetration depth into a workpiece to be irradiated and/or depending on the position of an in particular moving target volume to be irradiated of a workpiece to be irradiated

5. Method according to one of the preceding claims, in particular according to claim 4, **characterized in that** the position and/or the movement of an in particular moving target volume to be irradiated is determined in advance with an extrapolation algorithm.

6. Method according to one of the preceding claims, **characterized by** at least one verification means which verifies whether a required particle energy and/or a required change rate of the particle energy is still within the operating parameters of the particle accelerator device (1, 4).

7. Method according to one of the preceding claims, in particular according to one of claims 4 to 6, **characterized in that** a lateral change in the particle beam (23) generated by the particle accelerator device (1) is effected at least at times and/or at least in part in particular in the area of a target volume, and/or a one-dimensional, two-dimensional or three-dimensional scanning process (16) is conducted.

8. Particle accelerator device (1) for generating accelerated particles (23), comprising
a synchrotron with individual components arranged for a slow extraction, and
a control device (17, 18, 19, 20, 21, 31) to emit, process and/or receive a set energy signal at least at times and/or at least in part during a particle extraction phase (III),
where the control device (17, 18, 19, 20, 21, 31) has a storage device for storing equipment parameters depending on the set energy signal,
**characterized in that** the control device for controlling the individual components is set up such that the energy of the particles (23) released by the particle accelerator device (1) is changed (27) at least quasi-continuously at least at times (III) and/or at least in part during the particle extraction phase by changing (27) the energy of the particles (23) in the particle accelerator device (1).

9. Particle accelerator device (1) according to claim 8, **characterized by** at least one control device (17, 18, 19, 20, 21, 31), having at least one storage device in which equipment parameters are stored depending on a set energy signal, and/or having at least one calculating unit (17, 18, 19, 20, 21, 31) for interpolation and/or extrapolation of data stored in at least one storage device.

10. Control device for installation into a particle accelerator device (1), having a synchrotron with individual components arranged for a slow extraction, where the control device has at least one storage device for storing equipment parameters depending on a set energy signal, and/or having at least one storage device in which equipment parameters are stored depending on a set energy signal,
**characterized in that** the control unit is designed and set up such that the energy of the particles (23) released by the particle accelerator device (1) is changed (27) at least quasi-continuously at least at times (III) and/or at least in part during the particle extraction phase by changing (27) the energy of the particles (23) in the particle accelerator device (1).

11. Control device according to claim 10, **characterized in that** the control device has at least one calculating unit (17, 18, 19, 20, 21, 31) for interpolation and/or extrapolation of data stored in the storage device.

## Revendications

1. Procédé pour l'exploitation d'un dispositif d'accélération de particules (1) selon la revendication 8, qui présente un synchrotron avec différents composants disposés pour une extraction lente, **caractérisé en ce que** l'énergie des particules (23) libérées par le dispositif d'accélération de particules (1) pendant la phase d'extraction de particules est modifiée (27) en modifiant (27) l'énergie des particules (23) dans le dispositif d'accélération de particules (1) au moins quasi-continuellement, au moins temporairement (III) et/ou au moins partiellement.

2. Procédé selon la revendication 1, **caractérisé en ce qu'**il s'agit pour le dispositif d'accélération de particules (1) d'un dispositif d'accélération de particules pour hadrons, notamment pour protons, pour pions, pour ions et/ou pour ions lourds.

3. Procédé selon une des revendications précédentes, **caractérisé en ce que** les particules (23) sont accélérées au moins temporairement et/ou au moins partiellement en une première étape (I) à une valeur d'énergie initiale (E₀) et que dans une seconde étape (III), les particules sont accélérées et/ou retardées à une valeur d'énergie de consigne (Eᵢ).

4. Procédé selon une des revendications précédentes, **caractérisé en ce que** l'énergie des particules (23) libérées par le dispositif d'accélération de particules (1) est modifiée au moins temporairement et/ou au moins partiellement en fonction d'une valeur de consigne (Eᵢ) et/ou en fonction d'une valeur mesurée, de préférence en fonction d'une profondeur de pénétration requise dans une pièce d'oeuvre à irradier et/ou en fonction de la position d'un volume cible à irradier d'une pièce d'oeuvre à irradier, qui est notamment en mouvement.

5. Procédé selon une des revendications précédentes notamment selon la revendication 4, **caractérisé en ce que** la position et/ou le mouvement d'un volume à irradier, qui est notamment en mouvement, sont déterminés au préalable par un algorithme d'extrapolation.

6. Procédé selon une des revendications précédentes, **caractérisé par** au moins un moyen de vérification qui vérifie si une énergie de particules requise et/ou un taux de modification requis de l'énergie des particules se situe encore à l'intérieur de la plage des paramètres de service du dispositif d'accélération de particules (1, 4).

7. Procédé selon une des revendications précédentes, notamment selon une des revendications 4 à 6, **caractérisé en ce que** notamment dans la zone d'un volume cible, a lieu au moins temporairement et/ou au moins partiellement une modification latérale du faisceau de particules (23) généré par le dispositif d'accélération de particules (1) et/ou qu'est exécuté un procédé de balayage (16) unidimensionnel, bidimensionnel ou tridimensionnel.

8. Dispositif d'accélération de particules (1) pour générer des particules accélérées (23), comprenant
un synchrotron avec différents composants qui sont disposés pour une extraction lente, et
un dispositif de commande (17, 18, 19, 20, 21, 31) pour émettre, traiter et/ou recevoir un signal de consigne d'énergie au moins temporairement et/ou au moins partiellement pendant une phase d'extraction des particules (III),
sachant que le dispositif de commande (17, 18, 19, 20, 21, 31) présente un dispositif de stockage pour enregistrer des paramètres d'appareil en fonction de l'énergie de consigne d'énergie,
**caractérisé en ce que** le dispositif de commande servant à commander les différents composants est configuré de sorte à ce qu'en modifiant (27) l'énergie des particules (23) dans le dispositif d'accélération de particules (1) pendant la phase d'extraction des particules, l'énergie des particules (23) libérées par le dispositif d'accélération de particules (1) est modifiée (27) au moins quasi-continuellement, au moins temporairement (III) et/ou au moins partiellement.

9. Dispositif d'accélération de particules (1) selon la revendication 8, **caractérisé par** au moins un dispositif de commande (17, 18, 19, 20, 21, 31) qui présente au moins un dispositif de stockage dans lequel des paramètres d'appareil sont enregistrés en fonction d'un signal de consigne d'énergie, et/ou qui présente au moins une unité de calcul (17, 18, 19, 20, 21, 31) pour l'interpolation et/ou l'extrapolation des données enregistrées dans au moins un dispositif de stockage.

10. Dispositif de commande à monter dans un dispositif d'accélération de particules (1) qui présente un synchrotron avec différents composants disposés pour une extraction lente, sachant que le dispositif de commande présente au moins un dispositif de stockage pour enregistrer des paramètres d'appareil en fonction d'un signal de consigne d'énergie et/ou au moins un dispositif de stockage dans lequel des paramètres d'appareil sont enregistrés en fonction d'un signal de consigne d'énergie, **caractérisé en ce que** l'unité de commande est conçue et configurée de telle sorte que l'énergie des particules (23) libérées par le dispositif d'accélération de particules (1) est modifiée (27) au moins quasi-continuellement, au moins temporairement (III) et/ou au moins partiellement, par la modification de l'énergie des particules (23) dans le dispositif d'accélération de particules (1) pendant la phase d'extraction des particules.

11. Dispositif de commande selon la revendication 10, **caractérisé en ce que** le dispositif de commande présente au moins une unité de calcul (17, 18, 19, 20, 21, 31) pour l'interpolation et/ou l'extrapolation des données enregistrées dans le dispositif de stockage.
